(19) Europäisches Patentamt
European Patent Office
Office européen des brevets

(11) **EP 0 761 236 A2**

(12) **EUROPÄISCHE PATENTANMELDUNG**

(43) Veröffentlichungstag:
12.03.1997 Patentblatt 1997/11

(51) Int. Cl.⁶: **A61L 2/06**

(21) Anmeldenummer: 96114273.4

(22) Anmeldetag: 06.09.1996

(84) Benannte Vertragsstaaten:
AT BE CH DE FR IT LI NL

(30) Priorität: 09.09.1995 DE 19533443

(71) Anmelder: **Lobbe Umwelttechnik**
**58642 Iserlohn (DE)**

(72) Erfinder: **Straus, Reinhold**
**74821 Mosbach (DE)**

(74) Vertreter: **Fuchs, Luderschmidt & Partner**
**Abraham-Lincoln-Strasse 7**
**65189 Wiesbaden (DE)**

(54) **Vorrichtung zur Desinfektion und Trocknung von Vollschutzanzügen**

(57) Es wird eine Vorrichtung zur Desinfektion und Trocknung von Vollschutzanzügen (50) beschrieben, die kostengünstig ist und mit der beliebig große und beliebig ausgestattete Vollschutzanzüge auf einfache Weise gereinigt und zuverlässig getrocknet werden können. Die Vollschutzanzüge (50) werden über Kopf auf eine Hängevorrichtung aufgehängt, die ein verstellbares Rumpfteil (35) besitzt, an dem Armteile (11) und Beinteile (25) angeordnet sind. Diese Teile können ebenfalls verstellbar sein. Das Zuleitungsrohr (18) ist am Rumpfteil (35) über eine Drehdurchführung (17) angeschlossen und an einem fahrbaren Gestell (44) befestigt. Über einen Schlauch (14) wird Trocknungsluft in das Rumpfteil (35) eingeleitet und über die Arm- und Beinteile in die kritischen Bereiche, Zehen- und Fingerspitzenbereich, eingeleitet, wo sich die Austrittsöffnungen (41) für das Trocknungsmedium befinden.

FIG. 1

## Beschreibung

Die Erfindung betrifft eine Vorrichtung zur Desinfektion und Trocknung von Vollschutzarzügen gemäß dem Oberbegriff des Patentanspruchs 1.

Mit zunehmender Sensibilisierung der Bevölkerung in Richtung Umweltschutz werden auch in zunehmendem Maße Detektionsmessungen im Bereich der Chemie und Biologie durchgeführt. Nach den derzeitigen gesetzlichen Auflagen ist es erforderlich, daß die Vollschutzanzüge, die von dem Bedienungspersonal getragen werden, grundsätzlich nach jedem Einsatz gereinigt und desinfiziert werden, wobei die Vollschutzanzüge aufgrund der derzeitigen Materialbeschaffenheit nur bei Temperaturen um max. 60°C behandelt werden dürfen.

Aus der DE 3423436 C2 ist eine transportable Vorrichtung zum Dekontaminieren von Kampfanzügen bekannt, die auf einem Lastwagen angeordnet ist. Da die Dekontamination bei weitaus höheren Temperaturen, nämlich ca. 160°C, durchgeführt wird und entsprechend höhere Emissionen zu erwarten wären, werden auch höhere Anforderungen an die Vorrichtung gestellt. Es ist daher erforderlich, die Dekontamination innerhalb eines geschlossenen Raumes vorzunehmen. Innerhalb eines geschlossenen Behälters befindet sich eine Aufhängevorrichtung mit bügelähnlichen Gestellen, auf die die zu desinfizierenden Anzüge mit den Beinen nach unten aufgehängt werden. Über die nach oben offenen Bügel wird Heißluft zur Trocknung bzw. Dampf usw. in das Innere der Kampfanzüge eingeleitet.

In der DE 3421719 C2 werden die Kleiderbügel sowie deren Anschlußeinrichtung im einzelnen beschrieben.

Derartige Vorrichtungen sind für die Dekontamination einer großen Anzahl von Kampfanzügen ausgelegt und daher entsprechend teuer. Für die Reinigung einzelner. Anzüge kann eine solche Anlage unter Rentabilitätsgesichtspunkten nicht eingesetzt werden.

Bedingt durch die Aufhängung auf einem Kleiderbügel können nur solche Schutzanzüge gereinigt werden, die weder ein Kopfteil noch Stiefel bzw. Handschuhe aufweisen. Ein Kopfteil würde die Aufhängung des Bügels sowie die Zuführeinrichtung der Heißluft behindern. Eventuell vorhandene Stiefel und Handschuhe würden von den Düsen des bügelartigen Gestells nicht erreicht werden, so daß die erforderliche Trocknung im Bereich der Zehen- und Fingerspitzen nicht möglich wäre.

Um dieses Problem zu lösen, mußten in vielen Fällen die Anzüge gewendet werden, um einen möglichst schnellen Trockungseffekt zu erzielen. Die damit verbundene mechanische Belastung des Anzugmaterials und der personelle Aufwand waren entsprechend groß. Zur Abhilfe dieser Problematik wurden bereits die verschiedenartigsten Geräte und Anlagen auf den Markt gebracht.

Ein weiteres Problem besteht darin, unterschiedliche Anzuggrößen und -ausführungen, wie z.B. Länge 1,80 m oder Lange 2 m, Reißverschluß vorne links, Reißverschluß vorne rechts, Reißverschluß vorne Mitte bzw. Reißverschluß in Rückennähe, auf einer einzigen Vorrichtung reinigen und trocknen zu können.

Da in der Praxis tatsächlich die unterschiedlichsten Anzugvarianten zur Verwendung kommen, liegt die Hauptproblematik im Bereich der Halterungen für die jeweiligen Anzüge. Dies bedeutet, daß bisher nur Anzüge dekontaminiert werden können, für die geeignete jeweils angepaßte Halterungen vorhanden sind. Die auf dem Markt befindlichen Einfachvarianten, die für das Desinfizieren einzelner Anzüge gedacht sind, scheitern ebenfalls an der universellen Verwendbarkeit und haben einen hohen personellen Aufwand zur Folge.

Um diese Probleme zu lösen, wurde eine Vorrichtung entwickelt, mit der die Vollschutzanzüge über Kopf aufgehängt werden und die Trocknung in einem abgekapselten knapp bemessenen Raum durch Umpumpen von heißer Luft vorgenommen wird (Prospekt "Reinigung, Desinfektion, Trocknung" der Odenwaldwerke Rittersbach). Eine solche bekannte Vorrichtung weist ausziehbare Bein- und Armteile auf, um eine Anpassung an unterschiedlich große Vollschutzanzüge vornehmen zu können. Über ein Rumpfteil sind die Arm- und Beinteile miteinander verbunden. Bedingt durch den nur begrenzt zur Verfügung stehenden Tocknungsraum ist eine der Machart des Anzugs entsprechende knitterfreie Aufhängung nicht möglich.

Ferner bereitet das Aufhängen des Anzugs insofern Probleme, als der Bügel zum Überziehen eine eigene Vorrichtung benötigt, weil diese Arbeiten im Trocknungsraum aus Platzgründen nicht durchgeführt werden können.

Diese bekannte Vorrichtung hat weiterhin den Nachteil, daß die empfindlichen Bereiche, wie die Zehenspitzen des Vollschutzanzuges, vom Trocknungsmittel nur bedingt erreicht werden, weil die Austrittsöffnungen für das Trocknungsmittel im Beinbereich und nicht zehenspitzenorientiert angeordnet sind. Diese Vorrichtung läßt ferner nur eine begrenzte Anpassung an unterschiedlich große und verschieden ausgestattete Vollschutzanzüge zu.

Ausgehend von dieser bekannten Vorrichtung ist es Aufgabe der Erfindung, eine einfache, kostengünstige Vorrichtung zu schaffen, mit der beliebig große und beliebig ausgestattete Vollschutzanzüge, insbesondere geschlossene Vollschutzanzüge mit Stiefel- bzw. Handschuhen, auf einfache Weise gereinigt und zuverlässig getrocknet werden können.

Diese Aufgabe wird mit einer Vorrichtung gemäß den Merkmalen des Patentanspruchs 1 gelöst. Vorteilhafte Ausgestaltungen sind Gegenstand der Unteransprüche.

Dadurch, daß die Austrittsöffnungen für das Trocknungsmedium mindestens im Zehen- und Fingerbereich angeordnet sind, wird sichergestellt, daß für die Trocknung der empfindlichste Bereich mit der wärmsten und trockensten Luft beaufschlagt wird. Die Verstellbarkeit mindestens des Rumpfteiles in vertikaler Richtung gewährleistet eine Anpassung an unterschiedliche Größen der Schutzanzüge. Vorteilhafterweise sind auch die Arm- und/oder Beinteile ausziehbar, insbesondere teleskopierbar ausgebildet.

Das Zuleitungsrohr für das Trocknungsmedium ist einerseits über eine Drehdurchführung an das als Hohlteil ausgebildete Rumpfteil angeschlossen und andererseits außerhalb des Vollschutzanzuges am Gestell befestigt. Damit übernimmt das Zuleitungsrohr gleichzeitig auch eine Stützfunktion, weil das Rumpfteil mit den Arm- und Beinteilen und dem darüber aufgespannten Schutzanzug ausschließlich vom Zuleitungsrohr gehalten wird. Die Drehdurchführung ermöglicht durch ein einfaches Verschwenken des Zuleitungsrohres am Rumpfteil vorzugsweise um eine vertikale Achse, daß bei allen üblichen Vollschutzanzügen, die in vertikaler Richtung rechts, links oder Mitte den Reißverschluß aufweisen, das Zuleitungsrohr durch diesen Reißverschluß aus dem Vollschutzanzug herausgeführt werden kann, ohne daß Umbaumaßnahmen an der Vorrichtung vorgenommen werden müssen.

Vorteilhafterweise ist das Zuleitungsrohr auch an dem Gestell schwenkbar angeordnet. Das Zuleitungsrohr ist vorzugsweise am Rumpfteil um die Längsachse des Rumpfteils und am Gestell um eine zur Längsachse des Rumpfteils parallele Achse schwenkbar angeordnet. Durch dieses Doppelgelenksystem ist eine Z-förmige Verstellung des Zuleitungsrohrs möglich, so daß im wesentlichen die Ausrichtung des Schutzanzuges bezüglich des Gestells beibehalten werden kann. Dies ist insofern wichtig, als der Vollschutzanzug aus Stabilitätsgründen möglichst im Schwerpunkt des Gestells bzw. in dem Bereich des Gestells aufgehängt sein muß, der sich innerhalb des Bereichs der Auflageelemente des Gestells befindet. Das Gestell ist vorzugsweise transportabel, insbesondere fahrbar, so daß das Auf- und Abhängen der Anzüge in einem anderen Raum vorgenommen werden kann als z.B. das Reinigen, Desinfizieren und Trocknen. In dieser Ausführungsform werden als Auflageelemente Rollen verwendet.

Die Drehdurchführung mit dem Zuleitungsrohr kann oberhalb oder unterhalb der Armteile am Rumpfteil angeordnet sein, was im wesentlichen davon abhängt, ob der Reißverschluß des Vollschutzanzuges in Längs- oder in Querrichtung angeordnet ist. Quer angeordnete Reißverschlüsse können im Brust- oder Rückenbereich vorgesehen sein, so daß in diesem Fall das Zuleitungsrohr mit der Drehdurchführung unterhalb der Armteile angeordnet ist. Um diesen unterschiedlichen Reißverschlußanordnungen Rechnung zu tragen und um das Auf- und Abhängen des Vollschutzanzuges zu erleichtern, ist das Gestell vorzugsweise höhenverstellbar ausgebildet.

Die Arm- und Beinteile sind gemäß einer Ausführungsform als Rohre ausgebildet, deren Innenraum mit dem Hohlraum des Rumpfteils in Verbindung steht. Das durch das Zuleitungsrohr in das Rumpfteil eingeleitete Trocknungsmedium kann somit durch die Arm- und Beinteile in den Fingerspitzen- bzw. Zehenbereich geleitet werden, ohne daß zusätzliche Schläuche erforderlich sind. Die Arm- und Beinteile übernehmen somit die Funktion der Gasleitung und gleichzeitig auch die Funktion einer Aufspannvorrichtung für den Vollschutzanzug. Die Arm- und Beinteile können an das Rumpfteil angeschraubt sein.

Um die faltenfreie Aufhängung des Vollschutzanzuges noch weiter zu verbessern, besitzen die Beinteile an ihren freien Enden jeweils ein sich in den Stiefel des Vollschutzanzuges erstreckenden Fußabschnitt, der zum Aufspannen und Ausrichten der Stiefel mit Fußabstandshaltern versehen ist, was einen sicheren Auslauf aus dem Finger- und Zehenspitzenbereich ermöglicht. Auch die Armteile können an ihren freien Enden Handabstandshalter zum Aufspannen der Handschuhe aufweisen. Die Austrittsöffnungen für das Trocknungsmedium können am freien Ende des Fußabschnitts und/oder benachbart zu den Handabstandshaltern angeordnet sein. Durch die Kombination der Austrittsöffnungen im Handschuh- bzw. Stiefelbereich mit den dort angeordneten Abstandshaltern gibt es praktisch keine unzugänglichen Bereiche in diesen kritischen Abschnitten des Vollschutzanzuges, so daß eine rasche Trocknung ohne Umstülpung dieser Bereiche des Vollschutzanzuges möglich ist. Die Handabstandshalter können auch als Bügel ausgebildet sein.

Gemäß einer anderen Ausführungsform sind die Armteile stabförmig ausgebildet und tragen einen Gasschlauch, der an das Rumpfteil angeschlossen ist und im Bereich der Handabstandshalter endet und dort seine Austrittsöffnung aufweist. Dieser Schlauch ist vorzugsweise wegen der Verstellbarkeit der Armteile als flexibler Schlauch ausgebildet.

Da bei Vollschutzanzügen die Sauerstoffflaschen unter dem Anzug getragen werden müssen, sind im Rückenbereich des Anzuges entsprechende Ausbauchungen vorgesehen. Um auch in diesem Bereich das Ablaufen der Flüssigkeiten sicherzustellen, ist ein verstellbarer Rückenabstandshalter am Rumpfteil angeordnet.

Häufig werden Vollschutzanzüge bei der Detektion oder bei sonstigen Einsätzen im Chemiebereich speziell auf der Außenfläche stark kontaminiert. Bei der Reinigung bzw. Dekontamination kommt es in diesen Fällen sehr darauf an, daß sich keine Kontaminationen von außen in das Anzuginnere verlagern. Es ist daher erforderlich, daß der Reißverschluß des Anzugs vollständig geschlossen werden kann. Um dies zu erreichen, ist das Zuleitungsrohr vom Rumpfteil lösbar ausgebildet.

Das Gestell weist vorzugsweise eine Hängeeinrichtung mit Haltemitteln auf, die an den Beinteilen und/oder Fußabschnitten angreifen. Die Hängeeinrichtung weist vorteilhafterweise eine oberhalb des Zuleitungsrohres am Gestell angeordnetes Führungsstange auf, an der als Haltemittel zwei Bügel mit jeweils zwei Fingern zum Umgreifen der Beinteile und/oder der Fußabschnitte verschiebbar angeordnet sind.

Der Vollschutzanzug wird zunächst über die Fußabschnitte und Beinteile aufgehängt. Anschließend wird der Vollschutzanzug in die Hängeeinrichtung im Bereich der Fußabschnitte und/oder Beinteile eingehängt und das Zuleitungsrohr vom Rumpfteil gelöst. Beispielsweise durch Verschwenken des Zuleitungsrohres wird dieses nach dem Lösen vom Rumpfteil aus dem Bereich des Reißverschlusses entfernt, und der Reißverschluß kann anschließend verschlossen werden.

In diesem Zustand wird dann in der bereits beschriebenen Reihenfolge durch Vorreinigen, Aufsprühen und Einwirkenlassen von Desinfektionsmitteln sowie Nachreinigen die Außenreinigung vollzogen. Nach einer solchen Anzugaußendekontamination kann davon ausgegangen werden, daß von den weiteren Arbeiten keine Kontaminationsverlagerungen von außen nach innen stattfinden können. Das Abhängen des gereinigten Vollschutzanzuges erfogt dann in der umgekehrten Reihenfolge, indem zunächst das Zuleitungsrohr mit dem Rumpfteil befestigt wird und anschließend der Vollschutzanzug aus der Hängeeinrichtung entnommen wird.

Beispielhafte Ausführungsformen werden nachfolgend anhand der Zeichnungen näher erläutert.

Es zeigen:

Figur 1 eine Seitenansicht der Vorrichtung mit aufgespanntem Vollschutzanzug,

Figur 2 eine Frontansicht der im Inneren des Schutzanzuges angeordneten Bauteile der in Figur 1 gezeigten Vorrichtung,

Figur 3 eine Seitenansicht einer Vorrichtung mit aufgespanntem Vollschutzanzug gemäß einer weiteren Ausführungsform,

Figur 4 die im Inneren des Vollschutzanzuges befindlichen Bauteile der in Figur 3 gezeigten Vorrichtung in Frontansicht,

Figuren 5 und 6 Draufsichten auf zwei Ausführungsformen der Vorrichtung,

Figuren 7 und 8 eine Draufsicht auf die in Figur 6 gezeigte Vorrichtung in zwei verschiedenen Schwenkstellungen,

Figur 9 eine Seitenansicht der Vorrichtung mit aufgespanntem Vollschutzanzug und Besprühmöglichkeiten zur Innenreinigung sowie Wasserabführung im Kopfbereich,

Figur 10 die im Vollschutzanzug befindlichen Bauteile einer Vorrichtung gemäß einer weiteren Ausführungsform,

Figur 11 die in Figur 10 gezeigte Vorrichtung in Seitenansicht mit aufgespanntem Vollschutzanzug,

Figur 12 eine Detaildarstellung des Zuleitungsrohrs mit Drehdurchführung in Draufsicht,

Figuren 13, 14 Seitenansichten der Vorrichtung mit aufgespanntem Vollschutzanzug gemäß einer weiteren Ausführungsform und

Figur 15 die rückwärtige Ansicht der in den Figuren 13 und 14 gezeigten Vorrichtung.

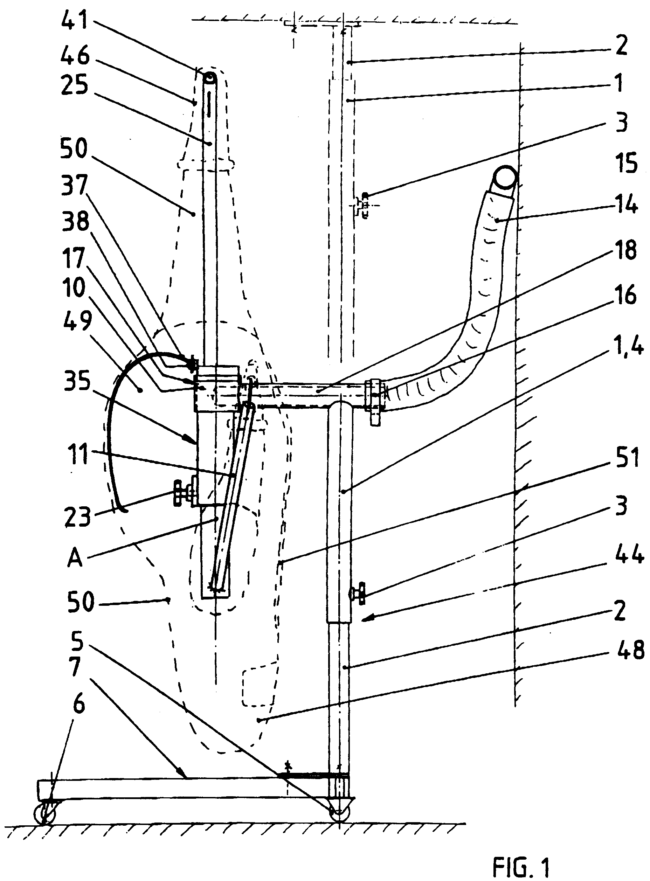

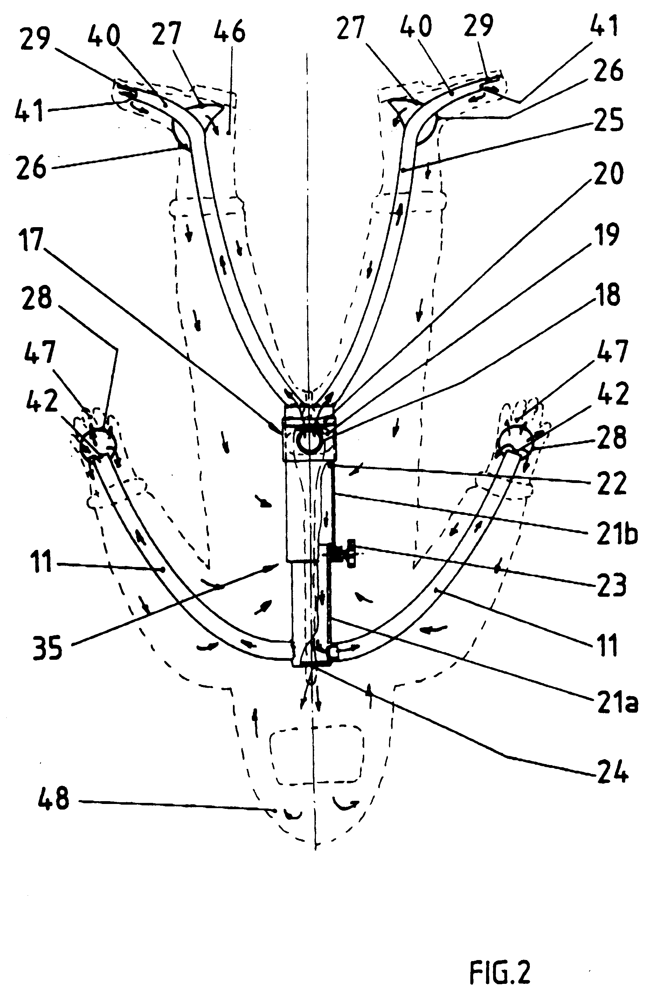

In den Figuren 1 und 2 ist eine erste Ausführungsform der Vorrichtung dargestellt. Der Vollschutzanzug 50 ist über Kopf faltenfrei aufgehängt. Es handelt sich hierbei um einen geschlossenen Vollschutzanzug mit Stiefel 46, Handschuhen 47 und Kopfteil 48. Im Inneren des Vollschutzanzuges 50 ist ein teleskopierbares Rumpfteil 35 vorgesehen, das zwei übereinandergesteckte Rohre 21a, 21b aufweist, die in vertikaler Richtung gegeneinander verschoben und mittels der Feststelleirrichtung 23 fixiert werden können. Die Anschläge 20 und 22 begrenzen den Verstellweg der beiden Rohre 21a, 21b.

Das Rumpfteil 35 ist hohl, so daß über den Rohrausschnitt 19 im Rohr 21b und die dort angeordnete Drehdurchführung 17 das Trocknungsmedium eingeleitet werden kann. Vom Rumpfteil 35 wird das Trocknungsmedium in die Armteile 11 und die Beinteile 25 geleitet, die in der hier gezeigten Ausführungsform ebenfalls als Rohre ausgebildet sind. Die Beinteile 25 sind am oberen Ende des Rohrs 21b angeordnet und sind zur Anpassung an die Beinkontur des Vollschutzanzuges 50 nach außen gekrümmt. Es besteht auch die Möglichkeit, die Beinteile gerade auszubilden. Im Bereich der Stiefel 46 setzen sich die Beinteile 25 in den Fußteilen 40 fort, die an ihrem freien Ende die Austrittsöffnungen 41 sowie Anschläge 29 aufweisen. Da sich Fußteile 40 bis in den Zehenbereich der Stiefel 46 erstrecken, kann das Trocknungsmedium auch in diesem Bereich austreten kann. Zum Aufspannen der Stiefel 46 sind Abstandshalter 26, 27 vorgesehen, die zusammen mit den Fußteilen 40 so ausgebildet und angeordnet sind, daß in allen Bereichen des Stiefels und der Beine des Vollschutzanzuges ein Gefälle vorhanden ist, so daß nach der Reinigung die Flüssigkeit nach unten ablaufen kann. Die Beinteile 25 sind daher schräg nach oben gerichtet, wobei die Fußabstandshalter 27 dornförmig ausgebildet sind und sich in den Fersenbereich des Stiefels 46 erstrecken. Die Beinteile 25 besitzen in der hier gezeigten Ausführungsform eine vorgegebene Länge, es ist jedoch auch möglich, die Beinteile ausziehbar zu gestalten.

Am unteren Ende des Rohes 21a des Rumpfteils 35 sind die Armteile 11 angeordnet, die ebenfalls als Rohre ausgebildet sind, so daß das Trocknungsmedium vom Rumpfteil 35 in den Bereich der Handschuhe 47 geleitet werden kann. Die Armteile 11 sind nach oben gebogen, so daß auch in den Handschuhen 47 und in den Armen des Vollschutzanzuges 50 ein Gefälle vorhanden ist, damit die Flüssigkeit auch hier nach unten ablaufen kann. Auch die Armteile können gerade ausgebildet sein. Dementsprechend sind auch die Handabstandshalter 28 am freien Ende der Armteile 11 so angeordnet, daß der Handschuh 47 aufgespannt ist. In der hier gezeigten Ausführungsform bestehen die Handabstandshalter 28 aus kreisförmig gebogenen Bügeln. Die Armteile 11 besitzen an ihrem freien Ende Austrittsöffnungen 42, durch die das Trocknungsmedium in den Fingerbereich der Handschuhe 47 ausströmt. Auch in diesem kritischen Bereich ist gewährleistet, daß das Trocknungsmedium hier zuerst ausströmt, bevor es die anderen Abschnitte des Vollschutzanzuges 50 erreichen kann.

Die Armteile 11 weisen eine vorgegebene Länge auf. Sie können auch ausziehbar ausgebildet sein, um eine bessere Anpassung an unterschiedliche Anzuggrößen vornehmen zu können. Damit das Trocknungsmedium auch ungehindert in den Kopfteil 48 des Vollschutzanzuges 50 austreten kann, ist im unteren Bereich des Rohres 21a des Rumpfteils 35 mindestens eine Austrittsöffnung 24 vorgesehen. Es können jedoch auch an beliebigen anderen kritischen Stellen Austrittsöffnungen für eine gleichmäßige Trocknung vorgesehen sein.

Ferner ist am Rumpfteil 35 in dem Bereich 49, wo beispielsweise ein Sauerstoffgerät unter dem Vollschutzanzug 50 getragen wird, ein Rückenabstandshalter 37 angeordnet, der zur Anpassung an unterschiedlich ausgestaltete Ausbauchungen des Vollschutzanzuges 50 verstellbar am Rumpfteil 35 angelenkt ist und mittels einer Feststelleinrichtung 38 fixiert werden kann.

Das in den Vollschutzanzug 50 gepumpte Trocknungsmedium durchströmt den gesamten Vollschutzanzug 50, so daß dieser wie ein Ballen aufgeblasen wird, und tritt dann durch den Reißverschluß 51 aus, der bei dem hier gezeigten Vollschutzanzug 50 vertikal angeordnet ist.

Wie in der Figur 1 zu sehen ist, besitzt die Drehdurchführung 17 eine Manschette 10, an der das Zuleitungsrohr 18 befestigt ist, das sich durch den Reißverschluß 51 nach außen erstreckt. Das Zuleitungsrohr 18 ist somit senkrecht zur Längsachse des Rumpfteils 35 angeordnet und um diese Längsachse A schwerkbar angeordnet.

An seinem freien Ende ist das Zuleitungsrohr 18 mit einem Schnellverschluß 16 an einen Schlauch 14 angeschlossen, durch den das Trocknungsmedium über eine Warmluftzuführung 15 in das Zuleitungsrohr 18 und somit in das Innere des Vollschutzanzuges 50 eingeleitet wird.

Das Zuleitungsrohr 18 ist an einem fahrbaren Gestell 44 befestigt, das ein äußeres Teleskoprohr 1 und ein inneres Teleskoprohr 2 aufweist, damit das Gestell höhenverstellbar ist. Mittels der Feststelleinrichtung 3 werden die beiden Rohre 1 und 2 gegeneinander fixiert. Das Rohr 2 ist auf einer fahrbaren Einrichtung 7 befestigt, die Rollen 5, 6 aufweist.

Innerhalb der Teleskoprohre 1 und 2 ist noch eine Rückstellfeder 4 vorgesehen, so daß beim Verstellen der Teleskoprohre 1, 2, was beim Aufhängen des Anzuges u.U. erforderlich ist, mit nur mäßigen Kräften wieder die vorgegebene Position eingenommen werden kann.

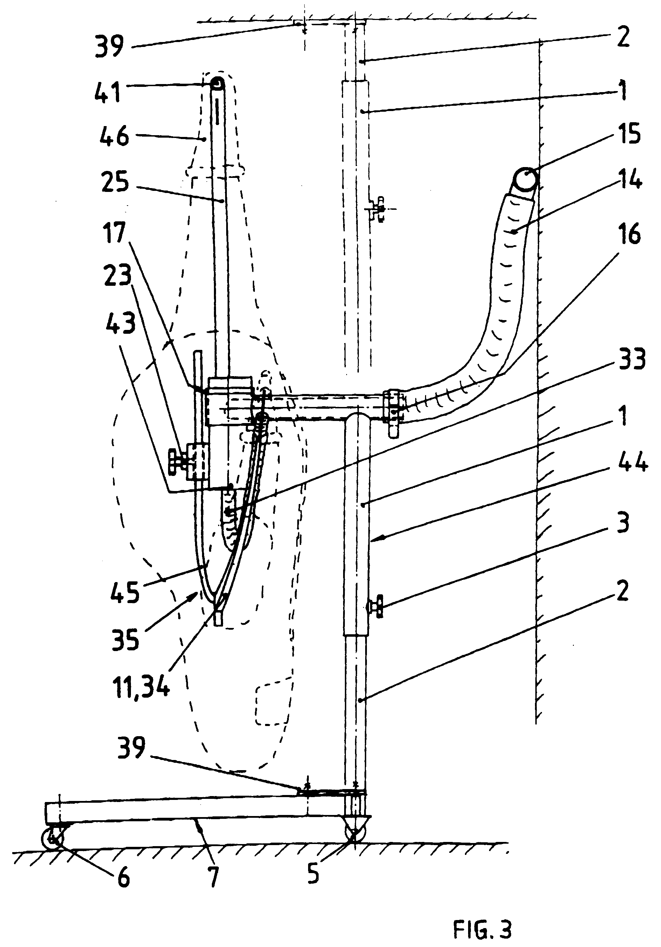

In den Figuren 1 und 3 ist als Alternative noch eine Deckenaufhängung gestrichelt dargestellt. Das Gestell 44 wird hierzu umgedreht und der Flansch 39 an der Decke befestigt. Das äußere Teleskoprohr 1 ist ebenfalls verschiebbar auf dem inneren Teleskoprohr 2 angeordnet.

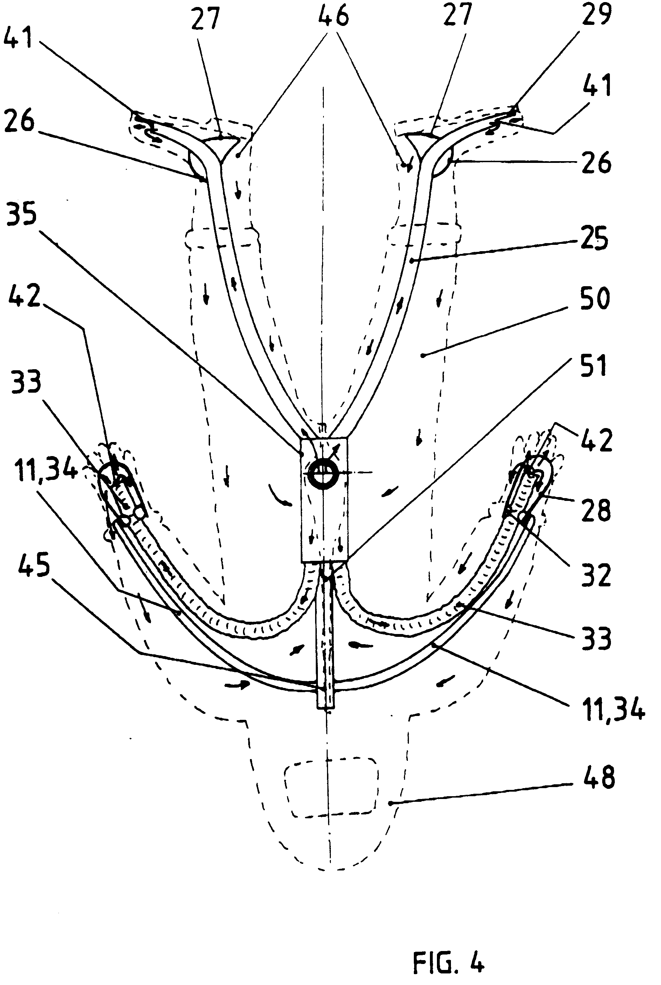

Die in den Figuren 3 und 4 dargestellte Ausführungsform unterscheidet sich von der Ausführungsform der Figuren 1 und 2 dadurch, daß die Armteile 11 als Stäbe 34 ausgebildet sind, an denen Schläuche 33 befestigt sind. Diese Schläuche 33 sind am Rumpfteil 35 angeschlossen und erstrecken sich bis in die Handschuhe 47, wo sich die Austrittsöffnung 42 befindet. Am freien Ende der Schläuche 33 bzw. der stabförmigen Armteile 34 sind Handabstandhalter 28 ebenfalls in Form von Bügeln angeordnet. Die Befestigung erfolgt über Halter 32, die verschiebbar an den Armteilen 11, 34 angeordnet sind, so daß ohne eine Veränderung der Armteile vornehmen zu müssen, eine Anpassung an unterschiedliche Armlängen möglich ist.

Das Rumpfteil 35 weist bei dieser Ausführungsform ein Rohr 43 auf, an dem das Zuleitungsrohr 18 über die Drehdurchführung 17 angeschlossen ist. Am Rohr 43 sind die Beinteile 25 und die Rohre 33 angeschlossen. Zusätzlich befindet sich an der Außenseite des Rohrs 43 eine Feststelleinrichtung 23, in der ein Stab 45 in Längsrichtung des Rumpfteils 35 verschieblich angeordnet ist. Der Stab 45 trägt am unteren Ende die Armteile 11, 34.

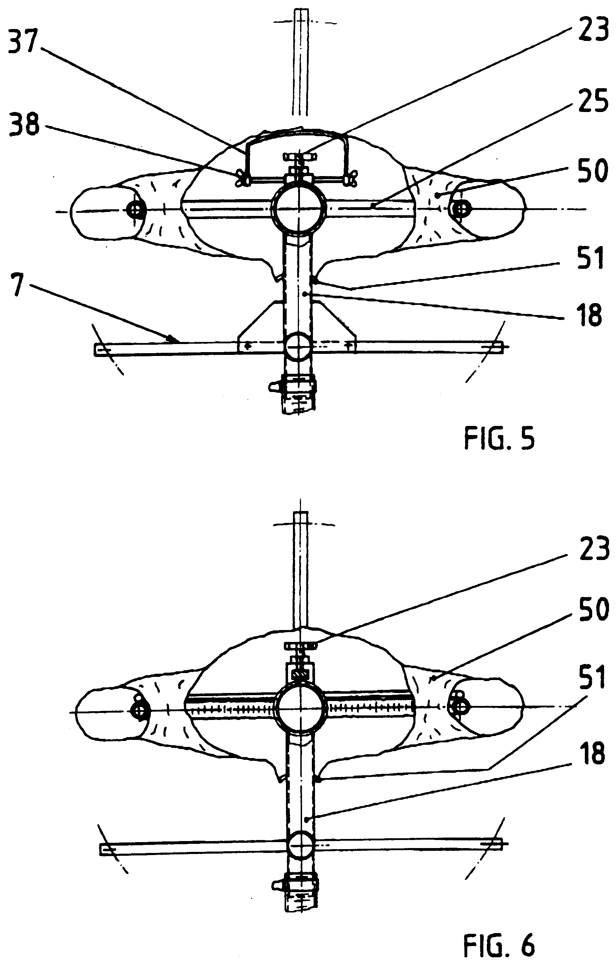

In den Figuren 5 bis 8 ist die Aufhängung verschiedener Vollschutzanzüge 50 dargestellt. Die Figuren 5 und 6 zeigen jeweils einen Vollschutzanzug 50 mit einem mittig angeordneten Reißverschluß 51. In der Figur 5 ist zusätzlich noch der Rückenabstandshalter 37 eingezeichnet. Da der Reißverschluß 51 mittig angeordnet ist, bildet das Zuleitungsrohr 18 im wesentlichen einen rechten Winkel zu den Beinteilen 25 und somit zur Querachse der Vollschutzanzuges 50.

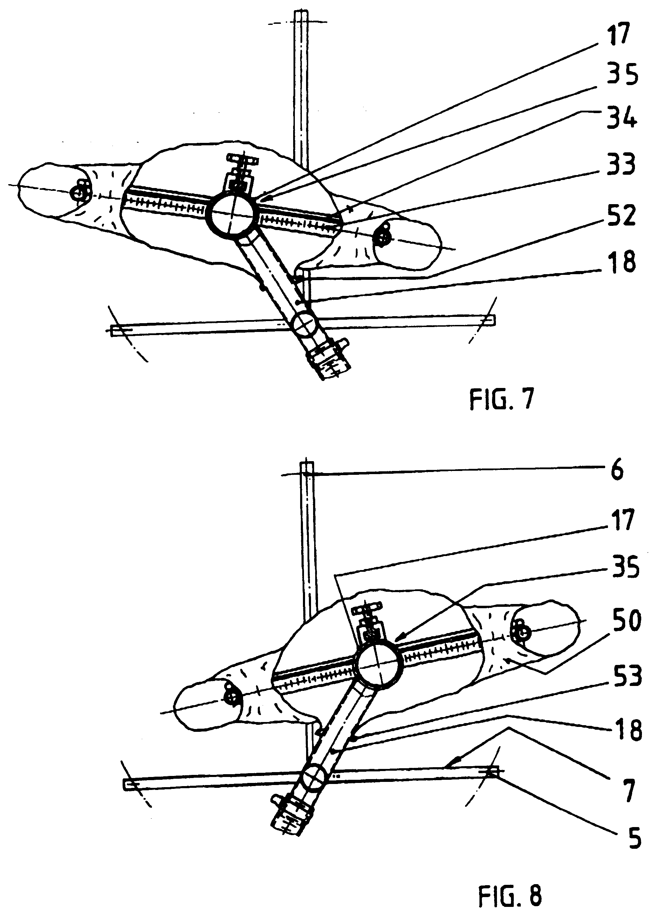

Wenn sich der Reißverschluß 52, 53 außermittig befindet, wie dies in den Figuren 7 und 8 dargestellt ist, wird über die Drehdurchführung 17 eine Z-förmige Ausrichtung des Zuleitungsrohres 18 gewählt. Hierbei ist es von Vorteil, wenn das Zuleitungsrohr 18 am Gestell 44, d.h. am Teleskoprohr 1 ebenfalls schwenkbar angeordnet ist. Die fahrbare Einrichtung 7 ist hierbei so dimensioniert, daß sich der Vollschutzanzug mit seinem Schwerpunkt trotz unterschiedlicher Ausgestaltung und Anordnung des Reißverschlusses aufgrund der Verschwenkmöglichkeiten des Zuleitungsrohres 18 in dem dreieckförmigen Bereich zwischen den Rollen 5 und 6 befindet.

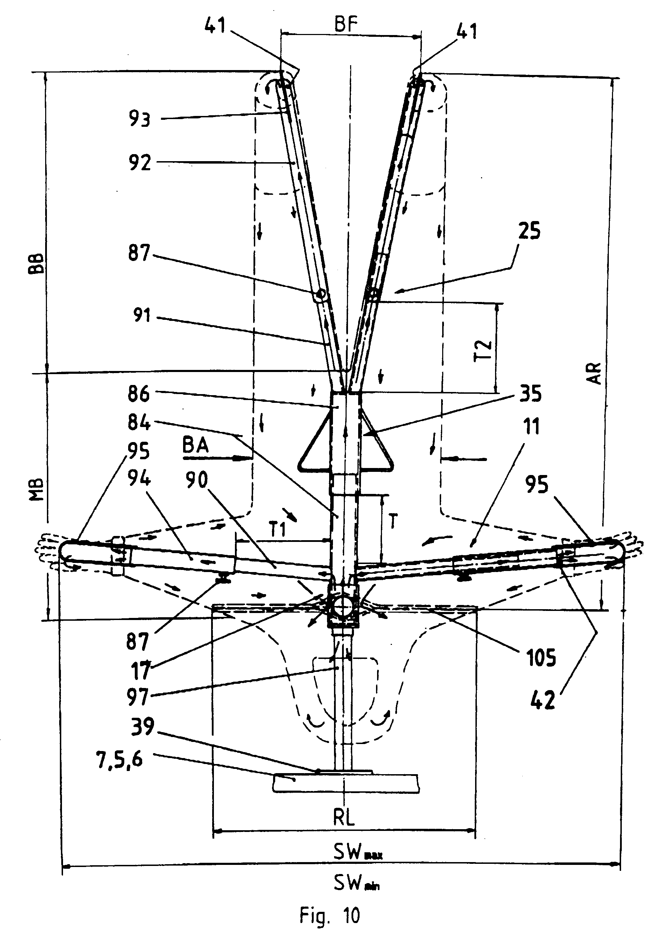

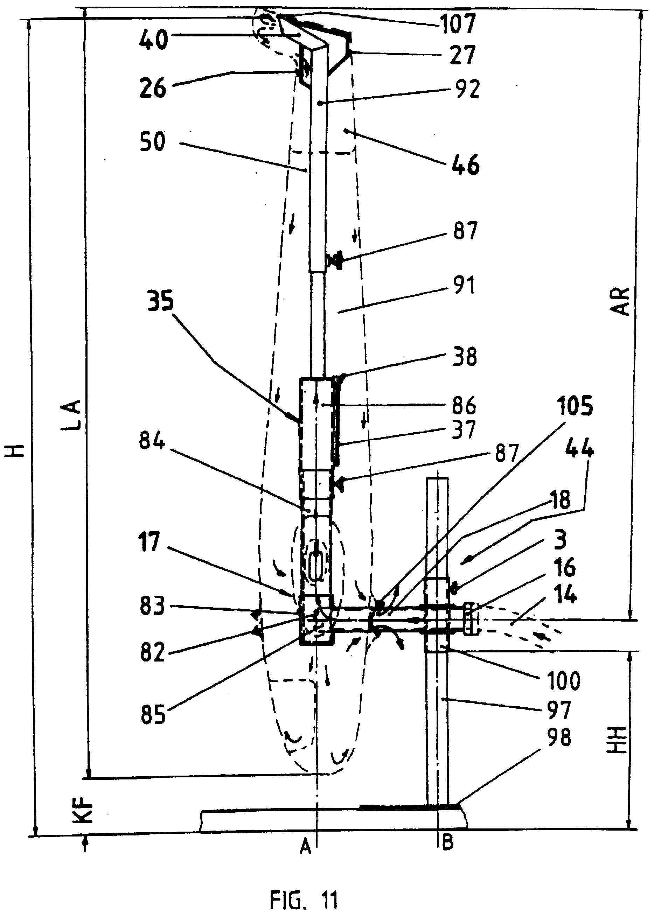

In den Figuren 10 und 11 ist eine weitere Ausführungsform dargestellt. Im Gegensatz zu den zuvor beschriebenen Ausführungsformen sind die Beinteile 25 teleskopierbar ausgebildet. Jedes Beinteil 25 besitzt zwei Führungsrohre 91, 92, die über eine Feststelleinrichtung 87 fixiert werden können. Die Führungsrohre 91 sind am Rumpfteil 35 befestigt. Am freien Ende der Führungsrohre 92 befinden sich die Austrittsöffnungen 41 für das Trocknungsmedium. Auch bei dieser Ausführungsform sind Fußabstandshalter 26, 27 vorgesehen, die so gestaltet sind, daß der Auflagepunkt 107 am vorderen Ende den höchsten Punkt bildet, so daß die Sohle des Stiefels geneigt ist (s. Fig. 11). Damit auch Vollschutzanzüge 50 mit im Brustbereich angeordneten Reißverschluß 105 aufgehängt und gereinigt werden können, sind die Führungsrohre 92 um ihre Längsachse drehbar ausgebildet. Der Anzug würde dann um 180°C verdreht aufgehängt werden und die Fußteile würden in diesem Fall zum Gestell 44 hinweisen. Der Verstellweg der Beinteile 25, 91, 92 ist durch das Maß T2 gekennzeichnet.

Das Rumpfteil 35 besitzt ebenfalls zwei verschiebbare Rohre 84, 86, wobei der Verstellweg mit T eingezeichnet ist. Im Gegensatz zu den zuvor beschriebenen Ausführungsformen befindet sich die Drehdurchführung 17 am unteren Ende des mit einem Rohrausschnitt 85 versehenen Führungsrohres 84, weil der Reißverschluß 105 horizontal im Schulterbereich des Vollschutzanzuges 50 angeordnet ist. Die Drehdurchführung 17 weist eine das Führungsrohr 84 umgreifende Manschette 83 auf, an der das Zuleitungsrohr 18 befestigt ist. Die Armteile 11 sind ebenfalls ausziehbar ausgestaltet und bestehen aus den Führungsrohren 90 und 94 mit den Austrittsöffnungen 42 sowie der Feststelleinrichtung 87 und den Handabstandshaltern. Das Verstellmaß ist mit T1 eingezeichnet.

Damit das Zuleitungsrohr 18 durch den Reißverschluß 105 geführt werden kann, ist es erforderlich, daß der Abstand AR realisiert werden kann, was zum einen durch die Verstellbarkeit des Rumpfteiles 35 und zum anderen durch die Verschiebbarkeit der Beinteile 25 ermöglicht wird. Da der Reißverschluß 105 im Schulterbereich angeordnet ist, muß die Drehdurchführung 17 möglichst weit unten angeordnet sein. Mit dieser Vorrichtung können auch Anzüge mit vertikalen Reißverschlüssen, die sich links, rechts oder in der Mitte befinden, aufgehängt werden, weil diese Anzüge diese Durchdringungsposition des Zuleitungsrohrs 18 überdecken. Mittels der Rohre 100 und 97 des Gestells 44 und mit Hilfe der Feststelleinrichtung 3 läßt sich das Maß HH im Rahmen der Verstellmöglichkeiten beliebig einstellen. So ist es z.B. möglich, beim Aufziehen des Anzuges das Maß HH bis zum unteren Anschlag hin zu verändern. Nach dem Aufziehen kann dann die Aufhängevorrichtung so weit nach oben verlagert werden, daß das nötige Kopffreimaß KF vorhanden ist.

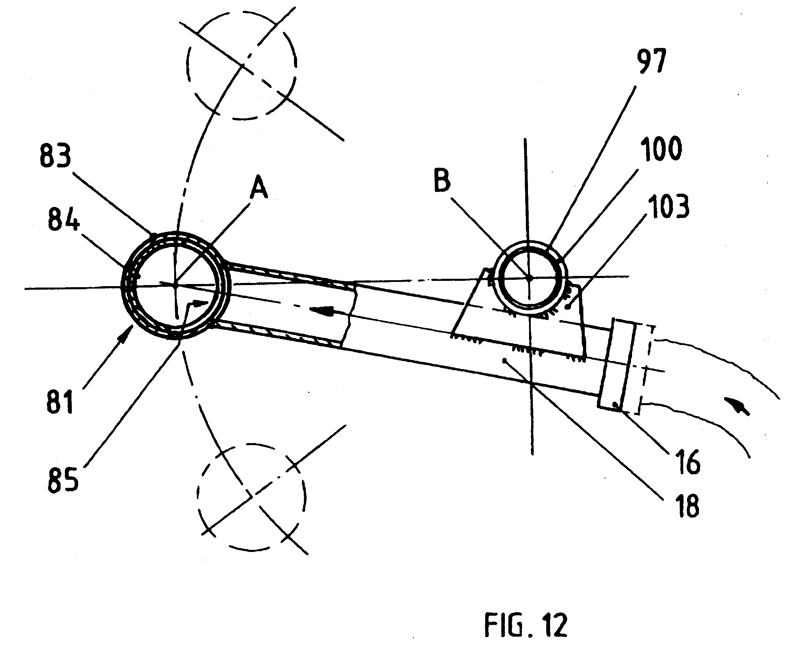

Zur Vereinfachung der Luftzuführung im Bereich des Führungsrohres 100 ist gemäß der Darstellung der Figur 12 das Luftzuführungsrohr 18 am Führungsrohr 100 vorbeigeleitet. Diese beiden Rohre sind mit Hilfe von Laschen 103 miteinander verbunden. Die Luft gelangt also mit Hilfe eines Schlauches 14 und einem Schnellverschluß 16 in das Luftzuführungsrohr 18 und von da über die Drehdurchführung 17 in das Führungsrohr des Rumpfteiles 35. Das Führungsrohr 84 des Rumpfteils 35 ist im Führungsrohr 83 drehbar gelagert und besitzt einen Rohrausschnitt 85. Mit Hilfe eines Anschlages 82 wird der Schwenkbereich der Drehdurchführung 17 begrenzt. Die Rohre 84 und 86 sind ineinander verschiebbar und stellen in Verbindung mit der Feststelleinrichtung 87 eine Teleskopiereinrichtung dar. Von diesem gemeinsamen Innenraum der Rohre 84 und 86 strömt dann die Luft wie bei den übrigen Ausführungsformen auch, einerseits über die Beinteile in den Stiefelbereich und andererseits über die Armteile in den Fingerspitzenbereich.

Um eine Einführung des Anzuges sicherzustellen, muß das Maß BF Kleiner sein als das Maß RL, das die Reißverschlußlänge bezeichnet. Ferner muß zur Bedingung gemacht werden, daß das Maß $SW_{max} - 2 \cdot T1 = SW_{min}$ eine solche Größenordnung erreicht, daß die beiden Ärmel eingeführt und danach durch Austeleskopieren der beiden Rohre 94 und entsprechender Feststellung mit Hilfe der Feststelleinrichtungen 87 eine annähernd faltenfreie Situation erreicht wird. Bedingt durch den begrenzten Mittenbereich MB ist nur ein begrenztes Teleskopiermaß T möglich. Eine weitere Teleskopier- und Feststellmöglichkeit besteht über T2. Dies ermöglicht wiederum die Einflußnahme auf den Beinlängenbereich. Aufgrund der Teleskopiermöglichkeit von T und T2 kann auf jede beliebige Anzuglänge sowohl im Bereich MB als auch im Bereich BB eingegangen werden. Dadurch ist in jeder Situation eine knitter- und faltenfreie Aufhängung möglich, was zu einem schnellen Trocknungseffekt führt.

Mit Hilfe einer solchen Vorrichtung ist es möglich, alle auf dem Markt befindlichen Vollschutzanzüge zu behandeln.

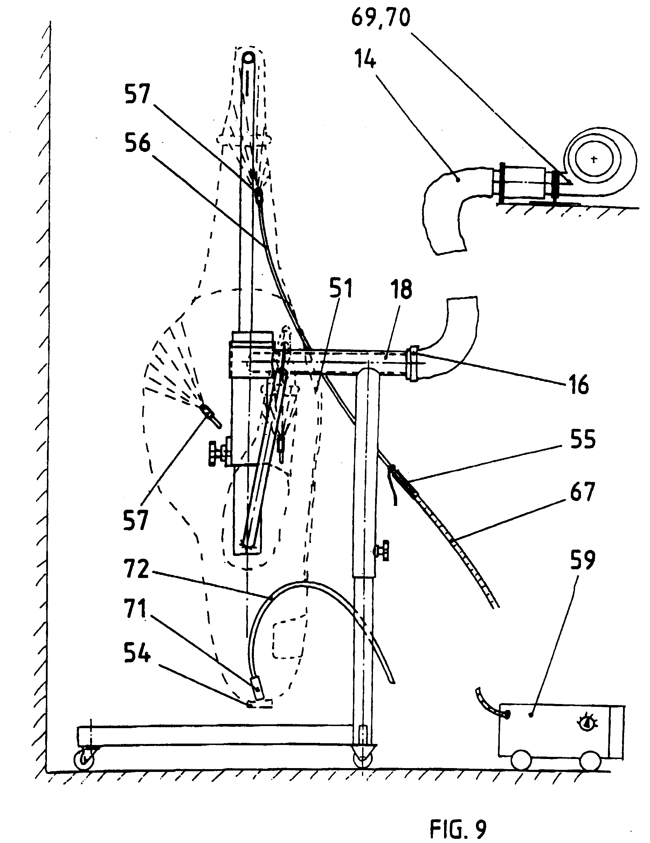

Das Desinfizieren und Trocknen eines Vollschutzanzuges wird nachfolgend anhand der Figur 9 erläutert. Der Vollschutzanzug 50 wird zunächst aufgehängt, wobei die Arm- und Beinteile 11, 25 zusammen mit den entsprechenden Abstandshaltern 26, 27 für ein faltenfreies Aufhängen des Vollschutzanzuges sorgen. Alle Teile des Vollschutzanzuges 50 werden so aufgehängt, daß immer ein Gefälle vorhanden ist, so daß die Flüssigkeit nach unten ablaufen kann.

Je nach Verschmutzungsgrad wird der Vollschutzanzug mit Kalt- oder Warmwasser und ggf. unter Zusatz von Reinigungsmitteln innen und außen gereinigt. Die Wasserzufuhr bei der Besprühung erfolgt über den Schlauch 67, das durch den Reißverschluß 51 einführbare Spezialstrahlrohr (Lanze) 56 mit Griffstück 55 und Handventil und über die Düse 57. Am Hochdruckreiniger 59 bzw. der Mischbatterie wird die Wassertemperatur so eingestellt, daß die zulässige Temperatur des Anzuges 50 nicht überschritten wird. Die Reinigung wird an einem Ort vorgenommen, an dem ein Wasserablauf sichergestellt ist.

Im nächsten Schritt wird der Vollschutzanzug desinfiziert. Hierzu wird dem Wasserzufluß Desinfektionsmittel mit Hilfe eines Beimpfungsgerätes eingeimpft und eine innen- und außenseitige Besprühung ebenfalls mit der Lanze vorgenommen. Danach erfolgt eine entsprechende Einwirkzeit und der Vollschutzanzug wird danach mit Wasser abgewaschen. Bei dieser Nachwäsche, die ebenfalls mit der Lanze 56 vorgenommen wird, kommt es in erster Linie darauf an, daß die Desinfektionsmittel bis in den Finger- und Zehenspritzenbereich abgewaschen werden. Hierfür ist es erforderlich, daß der Vollschutzanzug 50 so aufgehängt ist, daß ausreichend Platz zwischen den Arm- und Beinteilen und dem Vollschutzanzug zur Verfügung steht, um die Lanze 56 einführen zu können.

Zum anschließenden Trocknen des Vollschutzanzuges 50 wird mit Hilfe des Gebläses 70 die Luft durch ein Heizregister 69 gefördert und auf die für den Anzug maximal zulässige Temperatur erhitzt. Die so erwärmte Luft wird dann mit Hilfe des Schlauches 14 über das Zuleitungsrohr 18 und die Drehdurchführung 17 in das Rumpfteil 35 eingeleitet. Durch die zuvor beschriebene Anordnung der Austrittsöffnungen 41 wird die heiße Luft bis in den Finger- und Zehenspitzenbereich des Vollschutzanzuges 50 geleitet. Somit wird der für die Trocknung empfindlichste Bereich mit der wärmsten und noch trockenen Luft beaufschlagt. Die Luft strömt dann weiter über die Hosenbein- und Ärmelinnenseiten sowie den restlichen Innenbereich und entweicht durch den geöffneten Reißverschluß 51 ins Freie. Hierbei wird gleichzeitig das verdunstete Wasser nach außen befördert.

Bei Schutzanzügen 50 ohne Kopfventil 54 sammelt sich in der Regel in diesem Bereich Wasser an, welches nicht schnell genug durch Verdunsten abgeführt werden kann. In diesem Fall wird mit Hilfe einer zeitgetakteten Pumpe 71 und einem Schlauch 72 (s. Figur 9) das Wasser nach außen gepumpt. Dies kann auch durch Umstülpen des Anzugkopfbereiches oder durch Absaugen des Wassers mit Hilfe eines Schwammes erfolgen.

Der beschriebene Ablauf kann durch grobes Verschließen des Reißverschlusses 51 mit Hilfe von Klammern insofern begünstigt werden, daß dadurch bedingt im Innenraum des Anzugs 50 ein leichter Überdruck entsteht, was zu einer knitterfreien Aufhängung des Anzuges führt.

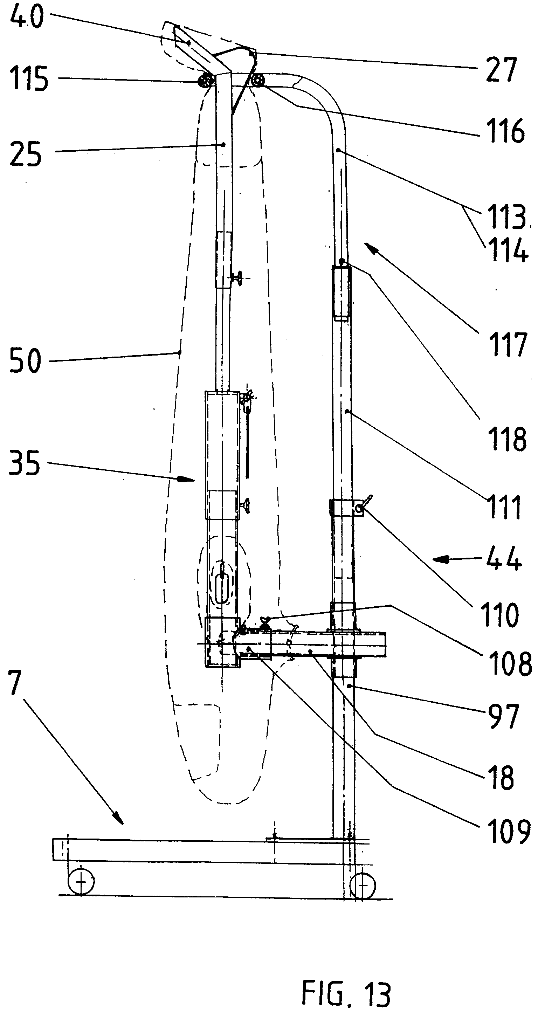

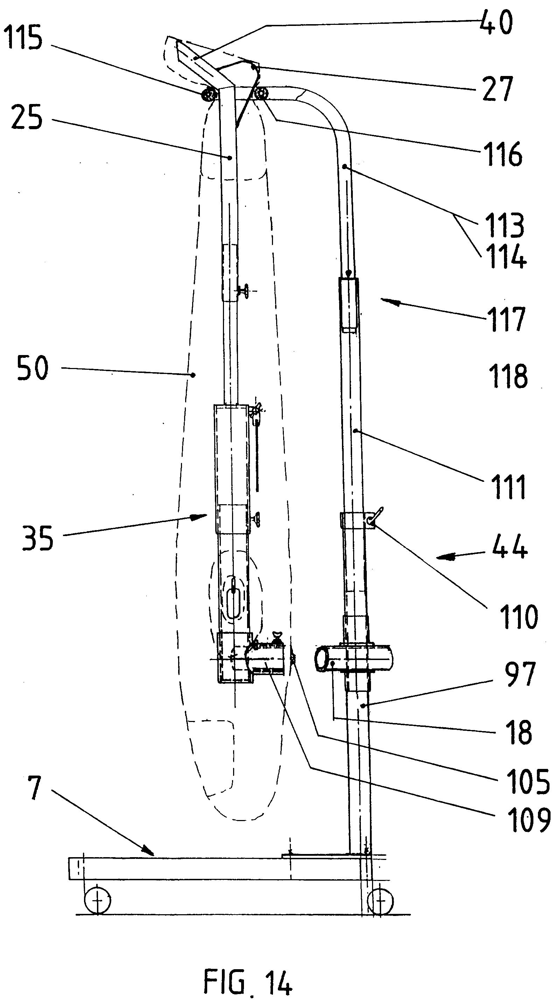

In den Figuren 13 bis 15 ist eine weitere Ausführungsform der Vorrichtung dargestellt, mit der die Verlagerung von Außenkontaminationen in das Anzuginnere verhindert werden kann.

Zunächst wird der Anzug gemäß den Figuren 10 und 11 aufgespannt. Da die Verbindung von Gestell 44 und Rumpfteil 35 über das Zuleitungsrohr 18 erfolgt, kann bei jeder Anzugart der Reißverschluß 105, 51, 52 und 53 nicht geschlossen werden. Um ein hermetisches Schließen des Vollschutzanzuges 50 realisieren zu können, ist das Zuleitungsrohr 18 in ein am Rumpfteil 35 befestigtes Rohr 109 einsetzbar und mit Hilfe einer Arretierung und Feststellvorrichtung 108 fixierbar. Vor der Trennung von Rumpfteil 35 und Gestell 44 wird der Anzug 50 zusammen mit der kompletten Rumpf-, Bein- und Armteilen aufweisenden Spannvorrichtung an einer Hängeeinrichtung 117 im Bereich der Fußabschnitte 40 aufgehängt.

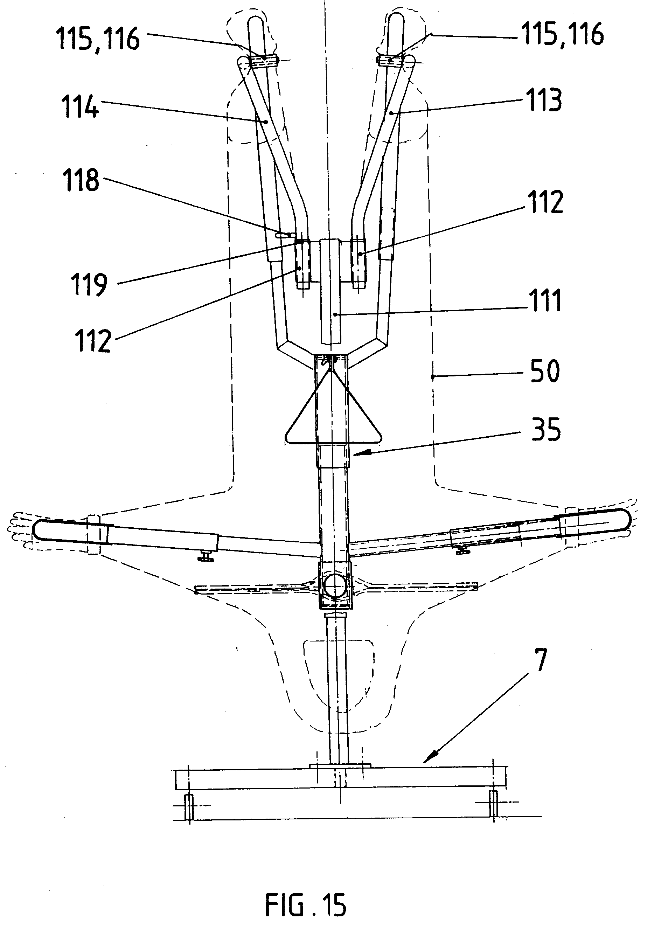

Die Hängevorrichtung 117 ist höhenvertellbar und kann mit Hilfe der Führungsstange 111 in das Führungsrohr 97 des Gestelles 44 einteleskopiert und mit Hilfe der Feststellungseinrichtung 110 in jeder beliebigen Höhe festgestellt werden. Am oberen Ende der Führungsstange 111 sind zwei Bügel 113, 114 in Lagerungen 112 schwenkbar angeordnet und mit einem Raststift 119 fixierbar. An ihrem freien Ende tragen die Bügel 113, 114 zwei sich in horizontale Richtung erstreckende Finger 115, 116, die mit einer Gummiauflage oder dergleichen zur Schonung des Anzuges überzogen sind (s. Figur 15). Die von rechts und links schwenkbaren Bügel 113 und 114 können mit ihren Fingern 115, 116 so in der Höhen- und Schwenkebene eingestellt werden, daß jede Art von Anzug bei jeder anzugspezifischen Gestelleinrichtung über den Abstandshalter 27 und den Fußabschnitt 40 aufgenommen werden kann. Das Schwenken der Bügel 113, 114 wird durch die Griffstücke 118 erleichtert.

Zur Stabilisierung der Hängeposition des Anzuges 50 sind die Finger 115 und 116 schräg eingebaut.

Die beschriebene Aufhängung funktioniert auch dann, wenn bei anderen Anzügen, die den Reißverschluß im Rückenbereich aufweisen, die Positionen 40 und 27 um 180° versetzt eingestellt werden müssen, d.h., wenn der Anzug um 180° verdreht aufgehängt werden muß.

Ist die beschriebene Einstellung erfolgt, kann die Abkoppelung des Zuleitungsrohres 18 vom Rohr 109 erfolgen und das Zuleitungsrohr 18, wie in Figur 13 gezeigt ist, geschwenkt werden.

Nach dem Schließen des Reißverschlusses ist der Vollschutzanzug bereit zur Außendekontamination. Die Ankoppelung erfolgt nach der Außendekontamination in umgekehrter Reihenfolge. Sollte beim Aufspannen des Anzuges die Hängeeinrichtung 117 hinderlich sein, können die Bügel 113, 114 bis 180° zurückgeschwenkt oder mit einem Handgriff ausgehoben werden. Ferner besteht die Möglichkeit, die komplette Einrichtung 117 bei abgeschwenkten Bügeln 113 und 114 abzusenken oder ebenfalls mit einem Handgriff auszubauen.

Bezugszeichen:

| | |
|---|---|
| 1 | Teleskoprohr außen |
| 2 | Teleskoprohr innen |
| 3 | Feststelleinrichtung |
| 4 | Rückstellfeder |

| | |
|---|---|
| 5 | Rolle |
| 6 | Rolle |
| 7 | fahrbare Einrichtung |
| 10 | Manschette |
| 11 | Armteil |
| 14 | Schlauch |
| 15 | Warmluftzuführung |
| 16 | Schnellverschluß |
| 17 | Drehdurchführung |
| 18 | Zuleitungsrohr |
| 19 | Rohrausschnitt |
| 20 | Anschlag |
| 21a,b | Rohr |
| 22 | Anschlag |
| 23 | Feststelleinrichtung |
| 24 | Austrittsöffnung |
| 25 | Beinteil |
| 26 | Abstandshalter |
| 27 | Abstandshalter |
| 28 | Abstandshalter |
| 29 | Anschlag |
| 32 | Halter |
| 33 | Luftschlauch |
| 34 | Stab |
| 35 | Rumpfteil |
| 37 | Rückenabstandshalter |
| 38 | Feststelleinrichtung |
| 39 | Flansch |
| 40 | Fußabschnitt |
| 41 | Austrittsöffnung |
| 42 | Austrittsöffnung |
| 43 | Rohr |
| 44 | Gestell |
| 45 | Stab |
| 46 | Stiefel |
| 47 | Handschuh |
| 48 | Kopfteil |
| 49 | Bereich |
| 50 | Vollschutzanzug |
| 51 | Reißverschluß mittig |
| 52 | Reißverschluß rechts |
| 53 | Reißverschluß links |
| 54 | Kopfventil |
| 55 | Griffstück mit Handventil |
| 56 | Spezialstahlrohr |
| 57 | Sprühdüse |
| 59 | Hochdruckreiniger |
| 67 | Wasserschlauch |
| 69 | Heizregister |
| 70 | Gebläse |
| 71 | Pumpe mit Zeittaktschaltung |
| 72 | Schlauch |
| 82 | Anschlag |
| 83 | Manschette |
| 84 | Führungsrohr innen |
| 85 | Rohrausschnitt |
| 86 | Teleskopohr |
| 87 | Feststelleinrichtung |
| 90 | Führungsrohr |

| | |
|---|---|
| 91 | Führungsrohr |
| 92 | Führungsrohr |
| 93 | Bügel |
| 94 | Führungsrohr |
| 95 | Handabstandshalter |
| 97 | Führungsrohr |
| 100 | Führungsrohr |
| 103 | Lasche |
| 105 | Reißverschluß quer |
| 107 | Auflagepunkt |
| 108 | Feststelleinrichtung |
| 109 | Rohr |
| 110 | Feststelleinrichtung |
| 111 | Führungsstange |
| 112 | Lagerung |
| 113 | Bügel |
| 114 | Bügel |
| 115 | Finger |
| 116 | Finger |
| 117 | Hängeeinrichtung |
| 118 | Griffstück |
| 119 | Raststift |

**Patentansprüche**

1. Vorrichtung zur Desinfektion und Trocknung von Vollschutzanzügen mit einer in das Innere des Anzugs greifenden und den Anzug tragenden Aufhängeeinrichtung, die Arm- und Beinteile zum Überkopfaufhängen des Vollschutzanzuges aufweist, die sich bis in den Bereich der Stiefel und Handschuhe des Vollschutzanzuges erstrecken, die Austrittsöffnungen für das Trocknungsmedium aufweist und die über ein Rumpfteil miteinander verbunden sind, das an ein Zuleitungsrohr für das Trocknungsmedium angeschlossen ist, welches außerhalb des Vollschutzanzuges an einem Gestell befestigt ist, dadurch gekennzeichnet,
daß mindestens im Finger- und Zehenbereich Austrittsöffnungen (41, 42) für das Trocknungsmedium angeordnet sind,
daß mindestens das Rumpfteil (35) ausziehbar ist und
daß das Zuleitungsrohr (18) über eine Drehdurchführung (17) an das als Hohlkörper ausgebildete Rumpfteil (35) angeschlossen ist.

2. Vorrichtung nach Anspruch 1, dadurch gekennzeichnet, daß das Zuleitungsrohr (18) am Rumpfteil (35) um die Längsachse A des Rumpfteiles (35) schwenkbar ist.

3. Vorrichtung nach einem der Ansprüche 1 oder 2, dadurch gekennzeichnet, daß das Zuleitungsrohr (18) schwenkbar am Gestell (44) angeordnet ist.

4. Vorrichtung nach Anspruch einem der Ansprüche 1 bis 3, dadurch gekennzeichnet, daß das Zuleitungsrohr (18) am Gestell (44) um eine zur Längsachse des Rumpfteiles (35) parallele Achse B schwenkbar ist.

5. Vorrichtung nach einem der Ansprüche 1 bis 4, dadurch gekennzeichnet, daß die Drehdurchführung (17) mit dem Zuleitungsrohr (18) oberhalb oder unterhalb der Armteile (11) am Rumpfteil (35) angeordnet ist.

6. Vorrichtung nach einem der Ansprüche 1 bis 5, dadurch gekennzeichnet, daß das Gestell (44) höhenverstellbar ist.

7. Vorrichtung nach einem der Ansprüche 1 bis 5, dadurch gekennzeichnet, daß das Gestell (44) fahrbar ist.

8. Vorrichtung nach einem der Ansprüche 1 bis 7, dadurch gekennzeichnet, daß die Arm- und/oder die Beinteile (11, 25) ausziehbar ausgebildet sind.

9. Vorrichtung nach einem der Ansprüche 1 bis 8, dadurch gekennzeichnet, daß die Arm- und Beinteile (11, 25) Rohre sind, deren Innenraum mit dem Hohlraum des Rumpfteiles (35) in Verbindung steht.

10. Vorrichtung nach einem der Ansprüche 1 bis 9, dadurch gekennzeichnet, daß die Beinteile (25) an ihrem freien Ende jeweils einen sich in den Stiefel (46) des Vollschutzanzuges erstreckenden Fußabschnitt (40) aufweisen, der mit Fußabstandshalter (26, 27) zum Aufspannen des Stiefels (46) des Vollschutzanzuges (50) versehen ist.

11. Vorrichtung nach einem der Ansprüche 1 bis 10, dadurch gekennzeichnet, daß die Armteile (11) an ihrem freien Ende Handabstandshalter (28) zum Aufspannen der Handschuhe (47) des Vollschutzanzuges (50) aufweisen.

12. Vorrichtung nach einem der Ansprüche 1 bis 11, dadurch gekennzeichnet, daß das Rumpfteil (35) einen Rückenabstandshalter (37) trägt.

13. Vorrichtung nach einem der Ansprüche 1 bis 12, dadurch gekennzeichnet, daß das Zuleitungsrohr (18) vom Rumpfteil (35) lösbar ausgebildet ist.

14. Vorrichtung nach einem der Ansprüche 1 bis 13, dadurch gekennzeichnet, daß das Gestell (44) eine Hängeeinrichtung (117) mit Haltemitteln aufweist, die an den Beinteilen (25) und/oder Fußabschnitten (40) angreifen.

15. Vorrichtung nach Anspruch 14, dadurch gekennzeichnet, daß die Hängeeinrichtung (117) ein oberhalb des Zuleitungsrohres (18) am Gestell (44) angeordnetes Führungsrohr (111) aufweist, an dem als Haltemittel zwei Bügel (113,114) mit jeweils zwei Fingern (115, 116) zum Umgreifen der Beinteile (25) und/oder der Fußabschnitte (40) verschiebbar angeordnet sind.

FIG. 1

FIG.2

FIG. 3

FIG. 4

FIG. 5

FIG. 6

FIG. 7

FIG. 8

FIG. 9

Fig. 10

FIG. 11

FIG. 12

EP 0 761 236 A2

FIG. 13

FIG. 14

FIG . 15